**Europäisches Patentamt**

**European Patent Office**

**Office européen des brevets**

(19)

(11) Veröffentlichungsnummer: **0 047 408**
**B1**

(12)

# EUROPÄISCHE PATENTSCHRIFT

(45) Veröffentlichungstag der Patentschrift:
**27.04.83**

(51) Int. Cl.³: **A 61 L 2/24**

(21) Anmeldenummer: **81106397.3**

(22) Anmeldetag: **18.08.81**

(54) Verfahren zum Entleeren, Reinigen, Desinfektion von Hygiene-Gefässen im Pflegesektor ohne Kontaminierung der äusseren Vorrichtung zu seiner Durchführung.

(30) Priorität: **19.08.80 CH 6248/80**

(43) Veröffentlichungstag der Anmeldung:
**17.03.82 Patentblatt 82/11**

(45) Bekanntmachung des Hinweises auf die Patenterteilung:
**27.04.83 Patentblatt 83/17**

(84) Benannte Vertragsstaaten:
**AT CH DE FR GB IT LI NL SE**

(56) Entgegenhaltungen:
**CH-A- 602 202**
**DE-A-2 712 020**

(73) Patentinhaber: **S I C AG, Wartenbergstrasse 15, Basel (CH)**

(72) Erfinder: **Harlegard, Jan, Dipl.-Ing., Adlerstrasse 32, CH-4052 Basel (CH)**

(74) Vertreter: **Jahn-Held, Wilhelm W., Dr.Dr.-Ing. Dipl.Chem., Schöne Aussicht 8, D-3513 Staufenberg 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Verfahren zum Entleeren, Reinigen, Desinfektion von Hygiene-Gefässen im Pflegesektor ohne Kontaminierung der äusseren Vorrichtung zu seiner Durchführung

Die Erfindung hat sich die Aufgabe gestellt, ein Verfahren zum Entleeren, Reinigen, Desinfizieren von Hygiene-Gefässen im Pflegesektor ohne Kontaminierung der äusseren Vorrichtung zu seiner Durchführung zu entwickeln.

Diese Aufgabe ergibt sich aus dem Oberbegriff des Patentanspruches 1.

Diese Aufgabe hat einen hohen volkswirtschaftlichen Wert, weil durch deren Lösung eine gesundheitlich und hygienisch einwandfreie Beseitigung von Abfallstoffen aus dem Pflegesektor möglich ist.

Aus der deutschen Offenlegungsschrift 29 38 695 ist bereits eine Anlage zur Reinigung und Desinfektion von Bettpfannen und deren Inhalt bekannt.

Diese Anlage nach dem Stand der Technik soll fahrbar sein. Im Gegenteil dazu ist die Vorrichtung der Erfindung stationär aufgestellt und an die Versorgungsleitungen und an die Ableitung angeschlossen.

Es soll nach dieser Veröffentlichung unter Druck eine Mischung eines Desinfektionsmittels mit Wasser in die Bettpfannen eingespritzt werden.

Das Verfahren der Erfindung verwendet gerade keine Desinfektionsmittel, sondern Wasser und Heissdampf. Die beiden Phasen des Wassers sind für das Verfahren der Erfindung ausreichend, da eine Kontaminierung der Vorrichtung durch Berühren mit den Händen vermieden wird.

Das Verfahren der Erfindung dreht zwar auch die in einem geschlossenen Spülraum eingebrachten Geräte, aber so, dass ein Herausspritzen des flüssigen oder festen Inhaltes auch bei rascher Drehung mit Sicherheit vermieden wird.

Das bekannte Verfahren unterscheidet sich wesentlich von dem Verfahren der Erfindung dadurch, dass eine Aufbewahrung der entleerten Bettpfannen über einen längeren Zeitraum in einem besonderen Schmutzwassertank notwendig ist. Ein derartiger gesonderter Lagertank ist für das Verfahren der Erfindung nicht notwendig. Dieses vermeidet auch eine Verzögerung der Durchsatzkapazität durch einen solchen, längeren, besonderen Desinfektionsvorgang mit Chemikalien und den damit verbundenen Arbeitsaufwand.

Ein wesentlicher Nachteil des bekannten Verfahrens liegt auch darin, dass dieses eine erhebliche Verdünnung des Inhaltes der zu reinigenden Gefässe wie Bettpfannen notwendig macht. Es müssen dadurch grössere Mengen an flüssiger Phase aus den Komponenten beseitigt werden. Die geleerte und desinfizierte Bettpfanne nach dem bekannten Verfahren ist auch nicht gebrauchsfertig. Es ist vielmehr notwendig, diese noch einem Spülvorgang zur Entfernung des anhaftenden Desinfektionsmittels und einem Trocknungsvorgang zuzuführen. Diese beiden Massnahmen sind in einer weiteren, getrennten Vorrichtung durchzuführen. Durch diese getrennte Behandlung wird bis zur Wiederbenutzung der Bettpfannen ein erheblicher Zeitaufwand notwendig.

Das Verfahren der Erfindung mit der Vorrichtung zu seiner Durchführung ist gerade einen anderen Weg gegangen, der die technischen Effekte der Erfindung nicht nahegelegt hat. Es wurde vielmehr dieser Stand der Technik endlich überwunden, den die Fachwelt noch bis kurz vor dem Verfahren der Erfindung mit seiner anderen Vorrichtung gegangen ist.

Auch die deutsche Offenlegungsschrift 2 938 400 benötigt für die Desinfektion von Schläuchen, Rohrleitungen und dergleichen ein flüssiges Desinfektionsmittel, das unter Druck in den zu desinfizierenden Hohlraum eingeführt wird. Die Desinfektion dieser an sich bereits leeren Gegenstände soll durch Einblasen von sterilem Gas in den Hohlraum eingeleitet werden. Eine derartige Behandlung ist für das Verfahren der Erfindung nicht erforderlich.

Dieses führt nach der Entleerung, eine Grobschmutzentfernung mit Kaltwasser, danach in weiteren Stufen eine Reinigung unter definierten Parametern mit Warmwasser, die Desinfektion mit Heissdampf unter gleichzeitiger Dampfkondensation durch. Erst die Kombination dieser Massnahmen führt zu einer kontinuierlichen, dynamischen Durchströmung des Spülraumes als Hohlraum mit dem Dampf und damit zu einer in kurzer Zeit mit optimal geringer Wassermenge erfolgenden Desinfektion. Danach erfolgt die Restkondensation und Rückkühlung der Gefässe und der Vorrichtung der Erfindung.

Nach dem Verfahren der Erfindung wird danach durch Tastendruck ohne Berührung der Vorrichtung mit den Händen zur Vermeidung einer Kontaminierung die Tür des Spülraumes geöffnet unter gleichzeitiger Rückdrehung der Befestigungshalterung mit den Gefässen zu deren Entnahme und zur Wiederholung der Verfahrensstufen. Das Verfahren der Erfindung ist mit seinem Lösungsweg und mit deren alternativer Ausgestaltung in den Patentansprüchen definiert.

Die Vorrichtung zu seiner Durchführung ist in dem daran anschliessenden Patentanspruch definiert.

Durch die Änderung der Drehungsgeschwindigkeit in Stufe (b) des Verfahrens der Erfindung wird der technische Effekt der raschen und vollständigen Entleerung ohne eine zusätzliche Zerkleinerung der fest-flüssigen Masse erreicht. Durch den besonderen Rotationseffekt in Stufe (c) wird eine rasche und praktisch vollständige Entfernung der Verunreinigungen und Reinigung der Flächen erreicht.

Unter «strahlungsgeometrischer» Verteilung des Druckwasserstrahles in Stufe (d) wird erfindungsgemäss die Aufteilung des Spülraumes in Sektoren durch gleichmässige Bedüsung aus den Sprühdüsen verstanden. Nur durch diesen technischen Effekt wird die kurzzeitige, vollständige Reinigung mit der optimal geringen Wassermenge erreicht.

Wesentlich ist für die Durchführung des Verfahrens der Erfindung die gleichzeitige Kondensation der eintretenden und den Spülraum durchströmenden Dampfmenge. Das Verfahren der Erfindung gestattet die eingeleitete und die austretende und zu kondensierende Dampfmenge so aufeinander abzustimmen, dass im Spülraum ein inneres Gleichgewicht herrscht, kein Überdruck auftritt, der Dampf durch die Abdichtung der Tür nach aussen führen könnte, oder auch kein grösserer oder überhaupt ein Unterdruck eintritt, der Luft von aussen durch die Abdichtung der Tür nach innen saugen könnte.

Die Parameter zur gleichmässigen Kondensation des Dampfes mit der Menge des Eintrittes, also mit der gleichen Menge des Wasserdampfes in der Zeiteinheit, sind Gegenstand der Erfindung.

Auch die Restkondensation von Dampfbrüden, die Rückkühlung des Spülraumes und der zu entnehmenden Gefässe kann als Verfahrensstufe mit den gleichen Massnahmen ohne eine zusätzliche Vorrichtung erfolgen.

Das Verfahren der Erfindung mit der Vorrichtung zu seiner Durchführung bietet den erheblichen Vorteil des raschen Durchsatzes an Geräten unter Vermeidung der Kontaminierung des Gerätes oder der gereinigten Geräte, da keine Berührung der Schalter mit den Händen bei der Einführung und Entnahme erfolgt.

Das Verfahen der Erfindung gestattet kurzzeitig mit optimal geringer Wasser- und Dampfmenge die Aufgabe der Erfindung zu lösen. Es sind keine Anschlüsse an besondere Geräte oder eine Entlüftung oder eine Fremdkondensation erforderlich.

Das Gesamtverfahren mit der Vorrichtung der Erfindung gestattet erstmalig durch die Reihenfolge und die Kombination der Massnahmen als technische Effekte die Lösung der Aufgabe der Erfindung als «in sich» arbeitendes Verfahren und Vorrichtung.

Das Verfahren der Erfindung arbeitet mit geringem Aufwand an Investierung, an Grösse und Umfang der Vorrichtung und an Energieverbrauch und an Wasser und Dampf. Die Beseitigung der Gefässinhalte erfolgt in bekannter Weise ohne zusätzliche Verdünnung.

Eine aus der schweizerischen Patentschrift 602 202 bekannte Vorrichtung zum Spülen und Desinfizieren von Steckbecken und dergleichen weist ein Gestell mit einer Spülkammer auf, die unten mit einem Ablauf verbunden ist und oben eine Öffnung aufweist, die mit einem durch einen Motor horizontal verschiebbaren Schiebedeckel verschliessbar ist. In der Spülkammer ist unter ihren oberen Rändern ein Ringkanal mit einem Auslass-Spalt vorhanden. Unter dem Ringkanal ist noch eine Anzahl Sprühdüsen verteilt angeordnet. Der Ringkanal ist über ein Elektroventil mit der Kaltwasser-Druckleitung des Gebäudes oder eventuell einem externen Spülkasten verbunden. Die Sprühdüsen sind über zwei Elektroventile mit der Kaltwasser- und Heisswasser-Druckleitung des Gebäudes verbunden. Bei einem der oberen Ränder der Spülkammer ist zwischen dieser und dem Deckel ein Spalt vorhanden, der den Innenraum der Spülkammer mit einer ausserhalb von dieser angeordneten Dampf-Kondensations-Vorrichtung verbindet. Diese weist eine mit einem Ablauf versehene Sammelrinne und ein in dieser entlang einem der beiden oberen Rinnen-Längsrändern verlaufendes Rohr auf, das auf seiner unteren Seite mit düsenartigen Austrittsöffnungen versehen ist.

Wenn in der aus der Schweizer Patentschrift 602 202 bekannten Vorrichtung ein Behälter zu reinigen ist, wird er durch eine Pflegeperson in die Spülkammer eingebracht. Dann betätigt die Pflegeperson einen Startschalter, der ein Programm-Steuer-Organ in Gang setzt. Dieses Steuer-Organ steuert nun den weitern Arbeitsablauf, so dass zuerst der Schiebedeckel die Spülkammer schliesst. Anschliessend wird in einer Vorspülphase durch den Ringspalt kaltes Wasser zugeführt, um die groben Schmutzteile wegzuspülen. Danach wird in einer Reinigungsphase während eines gewissen Zeitintervalls ein Gemisch von kaltem und warmem Wasser durch die Sprühdüsen zersprüht. In der darauf folgenden Desinfektionsphase wird durch die Sprühdüsen heisses Wasser mit einer Temperatur von ungefähr 95°C versprüht. Schliesslich wird nochmals ein Gemisch von kaltem und warmem Wasser zersprüht und anschliessend die Spülkammer wieder geöffnet. Da während der Desinfektionsphase Heisswasser zersprüht wird, entsteht in der Spülkammer eine beträchtliche Menge Dampf. Dieser kann durch den erwähnten Spalt in die Kondensations-Vorrichtung abströmen. In dieser werden mit dem Austrittsöffnungen aufweisenden Rohr eine Reihe von Kaltwasser-Strahlen erzeugt, die gewissermassen einen Vorhang bilden und den aus der Spülkammer ausströmenden Dampf kondensieren.

Die aus der Schweizer Patentschrift 602 202 bekannte Vorrichtung hat jedoch verschiedene Nachteile. Wenn beispielsweise in einem Spital ein von einem Patienten benutztes, Kot enthaltendes Steckbecken gereinigt werden soll, muss die Pflegeperson das Steckbecken zuerst manuell kippen und in die Spülkammer entleeren. Dabei besteht eine grosse Wahrscheinlichkeit, dass Mikroorganismen und eventuell sogar Kot-Teilchen auf die Haut oder Kleidungsstücke der Pflegeperson gelangen und auf diese Weise zu anderen Patienten verschleppt werden. Ein weiterer Nachteil der Vorrichtung besteht darin, dass sie im Grundriss relativ viel Platz beansprucht. Da nämlich der Deckel der Spülkammer beim Öffnen von dieser in horizontaler Richtung wegverschoben wird, muss die Vorrichtung eine in der Verschieberichtung des Deckels gemessene Länge aufweisen, die mindestens etwa das Zweieinhalb- bis Dreifache der in der gleichen Richtung gemessenen Spülkammerabmessung beträgt. Da bei der vorbekannten Vorrichtung der Ringkanal und die Sprühdüsen durch Leitungen direkt mit dem Leitungsnetz des Gebäudes verbunden sind, besteht zudem die Gefahr, dass bei einer etwa bei einer Leitungsreparatur erfolgenden Wasserrückströmung Verunreinigungen oder Mikroorganismen von der Spülkammer in das Leitungsnetz des Gebäudes gelangen können. Die direkte Speisung des Ringkanals und der Sprühdüsen aus dem Druckwasser-Leitungsnetz erfordert zudem, dass die Leitungsrohre zur Vermeidung eines allzugrossen Druckabfalles relativ grosse Querschnitte haben. Das ist um so mehr deshalb der Fall, weil in modernen Spitälern beispielsweise pro zwei bis zehn Betten eine Vorrichtung zum Spülen und Desinfizieren von Behältern vorhanden ist, so dass also unter Umständen eine grössere Anzahl solcher Vorrichtungen gleichzeitig benutzt wird. Es sei in diesem Zusammenhang noch bemerkt, dass zwar in der genannten Schweizer Patentschrift 602 202, wie bereits erwähnt, noch die Möglichkeit angeführt ist, den Ringkanal aus einem externen Spülkasten zu speisen. Ein derartiger, externer Spülkasten erfordert natürlich zusätzliche, externe Leitungen und müsste im übrigen zwangsläufig oberhalb der Spülkammer angeordnet werden. Da sich die Öffnung der Spülkammer auf deren oberer Seite befindet, würde ein Spülkasten, insbesondere wenn nur wenig Platz vorhanden ist, den Zugang zur Spülkammer erschweren. Schliesslich hat die vorbekannte Vorrichtung auch noch den Nachteil, dass sie für die mit Heisswasser erfolgende Desinfektion eine relativ grosse Menge Wasser erfordert und dass zu dessen Erhitzung auch eine entsprechend grosse Energiemenge benötigt wird. Des weitern wird auch zur Kondensation des Dampfes Wasser verbraucht.

Es sind auch noch Vorrichtungen zum Spülen und Desinfizieren von Steckbecken und dergleichen bekannt, bei denen eine mit Sprühdüsen versehene Spülkammer eine sich in der vertikalen Frontwand der Vorrichtung befindende Öffnung aufweist. Diese ist mit einer Türe verschliessbar, die manuell um eine horizontale, sich beim unteren Öffnungsrand befindende Schwenkachse verschwenkbar ist. Die Türe wird beim Öffnen in eine Stellung verschwenkt, in der sie horizontal nach aussen ragt. Die Türe ist im übrigen auf ihrer sich in offenem Zustand oben befindenden Seite mit Halterungen versehen, an denen Steckbecken und dergleichen fixiert werden können. Die Vorrichtung weist ferner einen oberhalb der Spülkammer angeordneten Wassertank auf, der über Zuleitungen mit dem Kalt- und Warmwasseranschluss des Gebäudes verbunden ist. Der Wassertank ist ferner über eine Pumpe mit den Sprühdüsen verbunden.

Bei der Benutzung dieser vorbekannten Vorrichtung wird ein zu reinigender Behälter bei geöffneter Türe auf einer entsprechend ausgebildeten Halterung fixiert. Danach wird die Türe zum Schliessen der Spülkammer manuell nach oben verschwenkt, wobei der Behälter gekippt und entleert wird. Anschliessend wird durch Betätigen eines Knopfes ein automatisch ablaufendes Arbeitsprogramm gestartet. Dabei wird zunächst in einer Vorspülphase mit der Pumpe kaltes Wasser aus dem Tank gepumpt und durch die Sprühdüsen in die Spülkammer gesprüht. In der darauffolgenden Reinigungsphase wird mittels der Pumpe warmes Wasser zu den Sprühdüsen gepumpt und durch diese zerstäubt. Danach findet noch eine Desinfektion statt, wobei diese je nach Ausführung der Vorrichtung mit Heisswasser, Dampf oder auf chemischem Weg erfolgen kann.

Bei dieser vorbekannten Vorrichtung wird die Türe zum Öffnen der Spülkammer nach aussen geklappt und ragt dann in horizontaler Richtung in den die Vorrichtung umgebenden Raum hinaus. Die Vorrichtung beansprucht daher bei offener Türe relativ viel Platz, was in vielen Fällen hinderlich ist. Dies ist insbesondere dann der Fall, wenn eine derartige Vorrichtung in einem Korridor oder sonstigen schmalen Durchgang aufgestellt werden muss. Man ist daher bei diesen Vorrichtungen häufig gezwungen, die Türe bei nicht benutzter Spülkammer geschlossen zu halten, so dass das Pflegepersonal die Türe zum Reinigen eines Behälters zuerst öffnen und nach dem Einsetzen des Behälters in eine Halterung wieder schliessen muss. Wenn also eine Pflegeperson beispielsweise ein Kot enthaltendes Steckbecken entleeren und reinigen will, muss sie die Türe mindestens ein Mal anfassen. Da die Pflegeperson in diesem Zeitpunkt häufig verschmutzte Hände hat, ist ein Anfassen der Türe aus hygienischen Gründen jedoch unerwünscht. Ein weiterer Nachteil dieser vorbekannten Vorrichtung besteht darin, dass beim Entleeren eines an der Türe befestigten Steckbeckens Kot oder Urin zu den Scharnieren oder auf die Aussenseite der Wände der Vorrichtung gelangen kann, was der Hygiene natürlich ebenfalls abträglich ist. Wie bereits erwähnt, wird sowohl das in der Vorspülphase verwendete Kaltwasser als auch das für die Reinigung dienende Warmwasser durch die Pumpe zu den Sprühdüsen gepumpt. Damit nun der Kot und andere Schmutzteilchen durch das beim Ablauf der Spülkammer vorhandene Syphon hindurchgespült werden, muss der Wasserdurchfluss pro Zeiteinheit in der Vorspüldüse einen gewissen Mindestwert haben. Die Pumpe muss daher derart konzipiert sein, dass sie eine entsprechende Förderleistung ergibt. Da man nun aber aus Platz- und Kostengründen eine möglichst kleine Pumpe mit geringem Energieverbrauch verwenden will, kann diese dann nur einen relativ kleinen Druck erzeugen. Insbesondere bei Zersprühen von warmem Wasser in der Reinigungsphase wäre es jedoch vorteilhaft, wenn das Wasser mit verhältnismässig grossem Druck gegen die zu reinigenden Behälter gesprüht wird.

Die vorliegende Erfindung hat sich auch zur Aufgabe gestellt, eine Vorrichtung zu schaffen, die einer Pflegeperson ermöglicht, einen etwa Fäkalien enthaltenden Behälter auf eine eine einwandfreie Hygiene gewährleistende Weise in die Spülkammer der Vorrichtung einzubringen und zu entleeren.

Diese Aufgabe wird durch die Vorrichtung zur Durchführung des Verfahrens der Erfindung gelöst, welche durch die Merkmale des Anspruches 10 gekennzeichnet ist.

Der Erfindungsgegenstand soll nun anhand eines Ausführungsbeispiels erläutert werden. In der Zeichnung zeigen

die Fig. 1 eine Frontansicht einer Vorrichtung zum Spülen und Desinfizieren,

die Fig. 2 eine Seitenansicht der Vorrichtung,

die Fig. 3 eine schematisierte, axonometrische Ansicht von gewissen Teilen der Vorrichtung und

die Fig. 4 ein Prinzip-Schema der Vorrichtung.

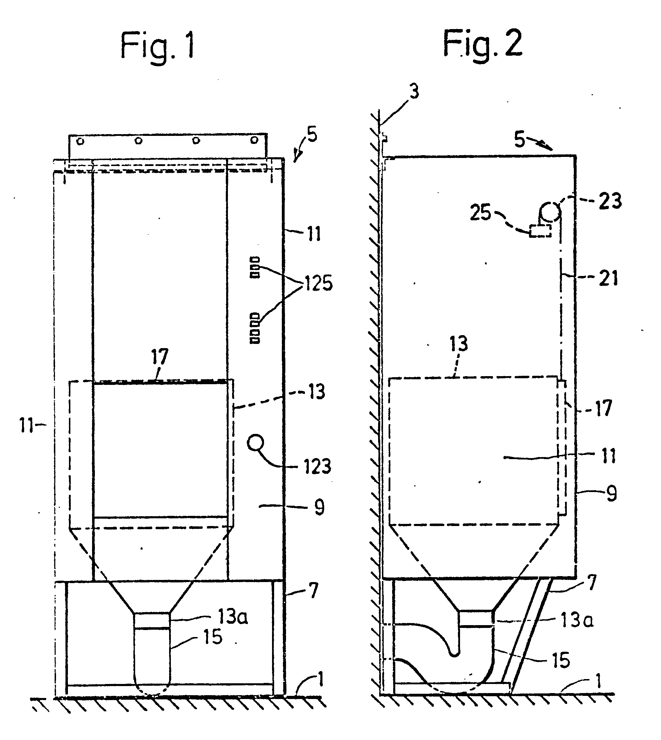

In den Fig. 1 und 2 sind der Boden 1 und eine Wand 3 des Rauems eines Gebäudes ersichtlich. Eine zum Spülen und Desinfizieren von Behältern dienende Vorrichtung 5 steht auf dem Boden 1 und ist zudem an der Wand 3 befestigt. Die Vorrichtung 5 weist ein Gestell 7 auf, an dem eine vertikale Frontwand 9 und zwei vertikale Seitenwände 11 befestigt sind, die zusammen einen Kasten bilden. In dessen unterem Teil ist ein Spülkasten 13 angeordnet und mit eine möglichst kleine Wärmeleitung ergebenden Befestigungsmitteln am Gestell 7 befestigt.

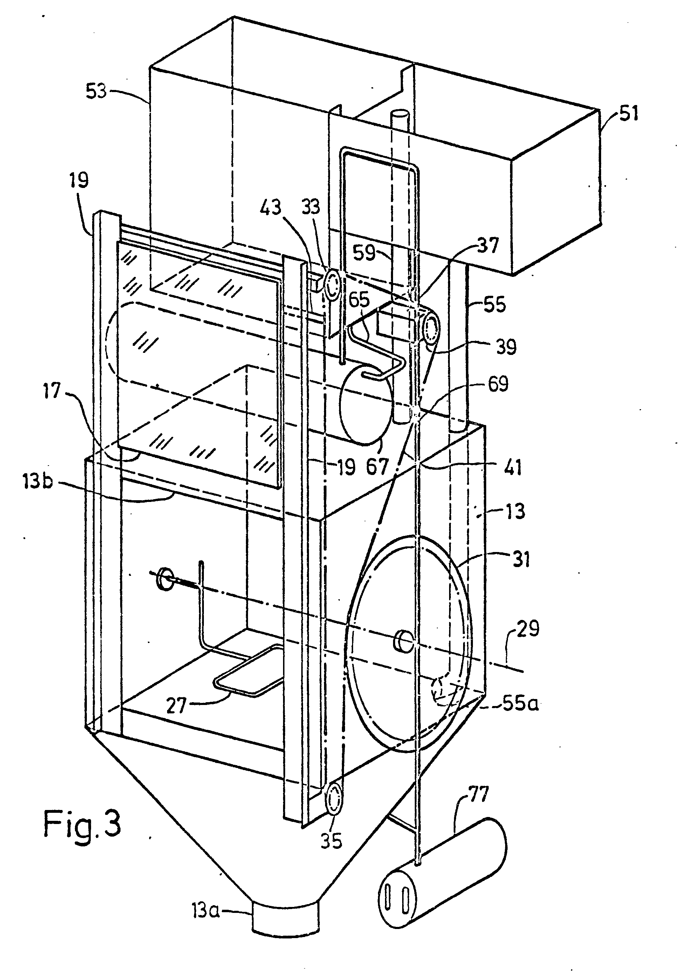

Die Spülkammer 13 weist oben einen quaderförmigen Teil auf, an den unten ein sich verjüngender Teil anschliesst, dessen unteres Ende den Ablauf 13a der Spülkammer bildet. Dieser ist über ein Syphon 15 mit der Ablaufleitung des Gebäudes verbunden. Der quaderförmige Teil der Spülkammer 13 ist auf deren Frontseite mit einer in der Fig. 3 ersichtlichen Öffnung 13b versehen. Diese ist mit einer Türe 17 verschliessbar, die in Führungen 19 entlang einer Vertikalebene auf- und abverschiebbar geführt ist. In ihrer unteren, in den Fig. 1 und 2 dargestellten Endstellung schliesst die Türe die Spülkammer 13 mindestens wasserdicht und vorzugsweise einigermassen dampfdicht ab, während sie diese in ihrer oberen, in der Fig. 3 dargestellten Endstellung freigibt. Am oberen Rand der Türe 17 ist ein nur in der Fig. 2 dargestelltes Seil 21 befestigt, das oberhalb der Türe bei einer Umlenkrolle 23 umgelenkt wird und an dessen anderem Ende ein Gegengewicht 25 befestigt ist.

In der Spülkammer 13 ist eine in der Fig. 3 dargestellte Halterung 27 angeordnet, die mit zwei Wellenzapfen um eine Schwenkachse 29 schwenkbar im Gestell bzw. den Seitenwänden der Spülkammer 13 gelagert ist. Die Schwenkachse 29 verläuft horizontal und parallel zur Vertikalebene, entlang der die Türe 17 verschiebbar ist. Die stark vereinfacht dargestellte Halterung 27 weist Mittel auf, um mindestens einen Behälter, beispielsweise ein Steckbecken und/oder eine Urinflasche derart zu halten, dass der Behälter auch beim Verschwenken der Halterung 27 nicht herausfällt. Im übrigen ist die Halterung zweckmässigerweise lösbar mit den zu ihrer Lagerung dienenden Wellenzapfen verbunden, so dass sie in einfacher Weise ausgewechselt werden kann, wenn dies etwa wegen der Verwendung anderer Behälter erforderlich sein sollte. Des weitern sind die Lager oder Durchführungen der Wellenzapfen in den Spülkammerwänden mindestens wasserdicht abgedichtet. Die Halterung 27 ist drehfest mit einem ausserhalb der Spülkammer angeordneten Kettenrad 31 verbunden. Zwei andere Kettenräder 33 und 35 sind beim oberen und unteren Ende der einen Führung 19 angeordnet. Ferner ist ein elektrischer Motor 37 mit einem Untersetzungsgetriebe und einem Kettenrad 39 vorhanden. Eine endlose Kette 41 ist derart um die Kettenräder 33, 35 und 39 herumgeführt, dass auch das Kettenrad 31 in sie eingreift. Der Motor 37 und das mit ihm verbundene Kettenrad 39 sind bewegbar im Gestell gehalten und durch eine nicht dargestellte Feder belastet, so dass das Kettenrad 39 die Kette 41 spannt. Zwischen den beiden Kettenrädern 33 und 35 weist die Kette 41 einen vertikal verlaufenden Abschnitt auf, der durch mindestens ein Verbindungselement 43 mit der Türe 17 verbunden ist. Im übrigen sind noch zwei nicht dargestellte Endschalter vorhanden, die betätigt werden, wenn die Türe ihre untere bzw. obere Endstellung erreicht.

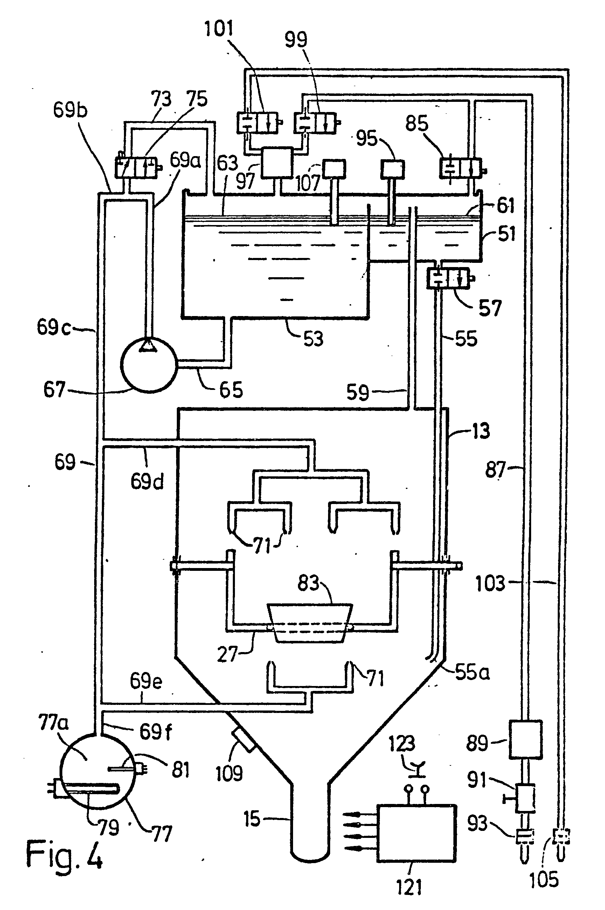

Oberhalb der Spülkammer 13 sind zwei Wassertanks 51 und 53 vorhanden, die bei einer Trennwand aneinander anstossen. Wie es in der Fig. 4 dargestellt ist, werden die beiden Tanks oben durch eine gemeinsame Decke abgeschlossen. Die erwähnte Trennwand reicht aber nicht oder mindestens nicht überall bis zur Decke. Die unterhalb des oberen Trennwand-Randes liegenden Füll-Niveaus der Tanks 51 und 53 sind mit 61 bzw. 63 bezeichnet. Zwischen den Füll-Niveaus und der Decke ist ein Zwischenraum vorhanden. Auch wenn die beiden Tanks bis zu ihren Füll-Niveaus gefüllt sind, ist über den Wasserspiegeln also noch ein Luft enthaltender Hohlraum vorhanden, wobei diese beiden Hohlräume bei der Trennwand zusammenhängen. Die beiden zusammenhängenden Hohlräume sind durch einen kleinen Durchgang mit der Umgebung verbunden.

Der Wassertank 51 ist bei seinem Boden mit einer vertikal nach unten verlaufenden Spül-Leitung 55 verbunden, die bei ihrer Mündung 55a in den Innneraum des Spülkastens 13 mündet. Die Mündung 55a befindet sich beispielsweise ungefähr beim Übergang zwischen dem quaderförmigen und dem sich verjüngenden Spülkastenteil und ist derart ausgebildet, dass das beim Spülen aus ihr ausströmende Wasser wirbelförmig entlang den Wänden des sich verjüngenden Spülkammerteils nach unten strömt. In die Spül-Leitung 55 ist ein elektrisch betätigbares Ventil 57 eingeschaltet, wie es aus der Fig. 4 ersichtlich ist. Ein als Dampf-Kondensator 59 dienendes Rohr führt von der Deckwand der Spülkammer 13 vertikal nach oben in den Wassertank 51 hinein, wobei sich die Rohrmündung oberhalb des Füll-Niveaus 61 befindet.

Der Wassertank 53 ist bei seinem Boden oder in dessen Nähe durch eine Leitung 65 mit dem Eingang einer sich unterhalb des Wassertanks 53 befindenden Pumpe 67 verbunden. Deren Ausgang ist durch eine Leitung 69 mit in der Spülkammer 13 vorhandenen Sprühdüsen 71 verbunden, die nur in der Fig. 4 schematisch dargestellt und derart angeordnet sind, dass sie Wasseroder Dampfstrahlen erzeugen können, die mindestens zum Teil gegen einen von der Halterung 27 gehaltenen Behälter 83 gerichtet sind. Die Leitung 69 weist einen vom Ausgang der Pumpe 67 vertikal bis über das Füll-Niveau 63 nach oben steigenden Abschnitt 69a auf. Der an diesen anschliessende, oberste und sich oberhalb des Füll-Niveaus 63 befindende Leitungs-Abschnitt 69b ist durch eine Entlüftungs-Leitung 73, in die ein elektrisch betätigbares Ventil 75 eingeschaltet ist, mit dem über dem Füll-Niveau 63 liegenden Innenraum des Tankes 53 verbunden.

An den horizontalen Leitungs-Abschnitt 69b schliesst ein vertikal nach unten verlaufender Abschnitt 69c an, von dem Abzweigungen 69d, 69e zu den Sprühdüsen 71 führen. Ein Wasser-Verdampfer 77 weist eine Kammer 77a auf, in die ein elektrisches Heizorgan 79 und ein Temperaturfühler 81 hineinragen. Die Kammer 77a ist unterhalb der untersten Sprühdüsen 71 und unterhalb des untersten, zu den Sprühdüsen führenden Abschnittes 69e der Leitung 69 angeordnet und durch den untersten Abschnitt 69f von dieser ebenfalls mit der Leitung 69 verbunden. Die Leitung 69 und der Wasser-Verdampfer 77 enthalten kein Ventil oder dergleichen, so dass also die Kammer 77a durch eine dauernd, d.h. in allen Arbeitsphasen offene Verbindung mit der Pumpe 67 und den Sprühdüsen 71 verbunden ist.

Der Wassertank 51 ist über ein elektrisch betätigbares Ventil 85 mit einer Kaltwasser-Speiseleitung 87 verbunden, die über ein Filter 89 und einen Hahn 91 mit einem Anschluss 93 des Kaltwasser-Druckleitungsnetzes des Gebäudes verbunden ist. Der Tank 51 ist ferner mit einem Niveaufühler 95 versehen.

Der Wassertank 53 ist mit dem Ausgang eines Mischventils 97 verbunden. Dieses weist zwei Eingänge auf, von denen der eine über ein elektrisch betätigbares Ventil 99 mit der Kaltwasser-Speiseleitung 87 und der andere über ein elektrisch betätigbares Ventil 101 mit einer Heisswasser-Speiseleitung 103 verbunden ist, die an einen Anschluss 105 des Heisswasser-Leitungsnetzes des Gebäudes angeschlossen ist. Der Wassertank 53 ist mit einem Niveaufühler 107 versehen.

Die Spülkammer 13 ist noch mit einem Temperaturfühler 109 versehen.

Des weitern ist ein elektrisches Programm-Steuerorgan 121 vorhanden, das mit einem Tastschalter 123 verbunden ist. Der letztere ist derart an der Frontwand 11 der Vorrichtung angeordnet, dass sein Druckknopf von einer Person mit dem Ellbogen betätigt werden kann. An der Frontwand 11 sind ferner einige Lampen und Tastschalter angeordnet, die in ihrer Gesamtheit mit 125 bezeichnet und mit dem Programm-Steuerorgan 121 verbunden sind. Diese ist ferner mit durch Pfeile angedeuteten, elektrischen Leitungen mit dem Motor 37, den mit der Türe 17 zusammenwirkenden Endschaltern, den elektrisch betätigbaren Ventilen 57, 75, 85, 99, 101, dem Motor der Pumpe 67, dem Heizelement 79, den Temperaturfühlern 81 und 109, den Niveaufühlern 95 und 107 sowie weiteren für die Betriebsüberwachung dienenden Elementen verbunden. Das Programm-Steuerorgan 121 ist mit den zur Steuerung und Überwachung des Arbeitsablaufes dienenden elektronischen Schaltungen, insbesondere Zeitgebern und Regelschaltungen sowie Schaltschützen versehen. Des weitern sind manuell einstellbare Stellorgane vorhanden, um gewisse Zeitintervalle sowie Temperatur-Sollwerte einzustellen.

Im folgenden sollen die Benutzung und der Betrieb der Vorrichtung 5 erläutert werden.

Wenn die Vorrichtung 5 betriebsbereit ist, aber nicht gerade zur Reinigung eines Behälters benutzt wird, befindet sich die Türe 17 in ihrer oberen Endstellung, in der sie die Öffnung 13b der Spülkammer 13 freigibt. Wenn nun eine Pflegeperson einen Behälter, etwa ein von einem Patienten benutztes, Kot enthaltendes Steckbecken, reinigen will, setzt sie diesen Behälter in die Halterung 27 ein, wie es in der Fig. 4 für den Behälter 83 dargestellt ist. Danach drückt die Pflegeperson mit dem Ellbogen auf den Druckknopf des Tastschalters 123. Dies bewirkt, dass das Programm-Steuerorgan 121 nun den Arbeitsablauf startet. Dabei wird zuerst der Motor 39 in Gang gesetzt, so dass dieser die Kette 41 bewegt und dadurch einerseits die Türe 17 in ihre untere Endstellung verschiebt, in der sie die Spülkammer-Öffnung 13b schliesst, und andererseits die Halterung 27 mit dem von dieser gehaltenen Behälter 83 um die Schwenkachse 29 verschwenkt. Das Kettenrad 31 ist derart bemessen, dass die Halterung 27 und der Behälter 83 um einen etwa 120 bis 180° betragenden Winkel verschwenkt werden und der Behälter 83 entleert wird. Wenn die Türe ihre untere Endstellung erreicht, was durch den erwähnten Endschalter zum Steuerorgan 121 zurückgemeldet wird, schaltet das letztere den Motor 39 wieder aus.

Während die Türe 17 geschlossen und der Behälter 83 gekippt wird oder wenn dieses geschehen ist, öffnet das Programm-Steuerorgan 121 das Ventil 57. Infolge der Schwerkraft strömt nun kaltes Wasser aus dem Wassertank 51 über die Spül-Leitung 55 in die Spülkammer und spült den aus dem Behälter 83 herausgekippten Kot durch das Syphon 15 hindurch weg. Nach einem vorgegebenen Zeitintervall, in dem der ganze Wasserinhalt des Tanks 51 abströmen kann und das beispielsweise 10 s beträgt, wird das Ventil 57 wieder geschlossen. Die Spül-Leitung 55 und das Ventil 57 sind dabei so bemessen, dass die pro Zeiteinheit zugeführte Wassermenge ausreichend gross ist, um die groben Schmutzteile durch das Syphon hindurchzuspülen. Je nach dem Durchmesser des verwendeten Syphons wird ein Mindestdurchfluss von 1 bis 2 l/s vorgesehen. Spätestens bei der Beendigung des Spülvorganges wird das Ventil 85 geöffnet, so dass der Tank 51 kurze Zeit nach der Beendigung der Spülphase wieder bis zum Füll-Niveau 61 gefüllt ist.

Ungefähr im Zeitpunkt, in dem die Türe geschlossen wird, setzt das Programm-Steuerorgan 121 auch die Pumpe 67 während eines vorgegebenen Zeitintervalls in Betrieb. Dieses Zeitintervall beträgt beispielsweise 30 bis 60 s und erstreckt sich also über das Ende der Spülphase hinaus. Die Pumpe 67 pumpt dann Wasser aus dem Tank 53 zu den Sprühdüsen 71, die das Wasser mindestens teilweise gegen den Behälter 83 sprühen. Dadurch wird der Behälter gespült und gereinigt. Während und nach dieser Reinigungsphase wird der Tank 53 über die elektrisch betätigbaren Ventile 99 und 101 sowie das Mischventil 97 nachgefüllt. Das ein thermo-mechanisches Regelelement aufweisende Mischventil 95 ist so beschaffen, dass der Tank mit einem Wassergemisch nachgefüllt wird, dessen Temperatur ungefähr 40 bis 45°C beträgt. Wenn die Vorrichtung vorher lange Zeit nicht benutzt worden war, hat das beim Beginn der Reinigungsphase aus dem Tank 53 herausgepumpte Wasser Raumtemperatur. Während des schon während des Abpumpvorganges erfolgenden Nachfüllens des Tanks steigt dann die Wassertemperatur bis auf die erwähnten 40 bis 45°C an. Das von den Sprühdüsen 71 zersprühte Wasser hat also eine höchstens 40 bis 45°C betragende und damit unter der Koagulationstemperatur von Eiweiss liegende Temperatur. Die Pumpe 67 erzeugt bei den Sprühdüsen einen Druck, der wesentlich grösser ist, als der durch die Schwerkraft infolge der Höhendifferenz erzeugte Druck, so dass das von ihr gepumpte Wasser mit einer Geschwindigkeit aus den Sprühdüsen austritt, die wesentlich grösser ist als diejenige des beim Vorspülen aus der Mündung 55a der Spül-Leitung 55 ausströmenden Wassers, hingegen ist die von der Pumpe 67 pro Zeiteinheit geförderte Wassermenge kleiner als die beim Vorspülen durch die Spül-Leitung 55 pro Zeiteinheit zugeführte Wassermenge und beträgt beispielsweise 0,5 bis 1 l/s.

Während des Betriebes der Pumpe 67 ist das Ventil 75 geschlossen und sperrt die Leitung 73. Wenn jedoch das Steuerorgan 121 die Pumpe 67 am Ende der Reinigungsphase wieder ausgeschaltet hat, betätigt es kurzzeitig das Ventil 75. Dadurch wird die Leitung 69 vorübergehend mit den in den beiden Tanks 53, 55 über den momentanen Wasserspiegeln vorhandenen Hohlräumen verbunden, die, wie bereits erwähnt, mit der Umgebung verbunden sind und also unter Umgebungsdruck stehen. Beim Öffnen des Ventils 75 wird daher die Leitung 69 bei ihrem obersten Abschnitt 69b belüftet. Das sich im Leitungs-Abschnitt 69c befindende Wasser strömt dann bis zum Niveau der tiefsten Sprühdüse durch die Sprühdüsen aus. Da sich der Wasser-Verdampfer 77 unterhalb der untersten Sprühdüsen befindet, bleiben also der unterste Teil der Leitung 69 und insbesondere der Wasser-Verdampfer bei der Entlüftung mit Wasser gefüllt. Da die Pumpe 67 im Stillstand die Leitung 69 nicht dicht absperrt, bleibt dagegen das Wasser in dem sich zwischen der Pumpe 67 und dem Leitungs-Abschnitt 69b befindenden Leitungs-Abschnitt 69a gleich hoch stehen wie im Tank 53. Weil sich der Leitungs-Abschnitt 69b über dem Füll-Niveau 63 befindet, kann bei abgeschalteter Pumpe kein Wasser aus dem Tank 53 abströmen.

Während die Pumpe 67 Wasser zu den Sprühdüsen pumpt, schaltet das Programm-Steuerorgan 121 schon das Heizorgan 79 ein. Ungefähr eine halbe Minute nach dem Abschalten der Pumpe erreicht das Wasser im Verdampfer 77 Siedetemperatur und beginnt zu verdampfen und als Dampf durch die Sprühdüsen in die Spülkammer 13 zu strömen. Durch diesen Dampf wird der sich in der Spülkammer befindende Behälter 83 desinfiziert. Mit dem Temperaturfühler 109 wird dabei die Temperatur an der Spülkammerwand oder in der Spülkammer gemessen. Die Dampfzufuhr erfolgt so lange, dass die vom Temperaturfühler 109 gemessene Temperatur während eines vorgegebenen Zeitintervalls von beispielsweise 30 bis 120 s einen vorgegebenen Mindestwert aufweist, der beispielsweise 83 bis 90°C beträgt.

Wie bereits erwähnt, bleibt nach dem Abschalten der Pumpe 67 und dem Entlüften der Leitung 69 in deren Abschnitt 69a und auch in der Pumpe und Leitung 65 Wasser stehen. Diese Leitungsstücke bilden also zusammen mit dem Wassertank 53 eine Art Syphon, das als Dampfsperre wirkt und verhindert, dass der im Verdampfer 77 erzeugte Dampf über die Pumpe in den Wassertank abströmt.

Der in der Desinfektionsphase zugeführte Dampf kann in den Dampf-Kondensator 59 hineinströmen. Da dieser durch das im Tank 51 vorhandene, kalte Wasser gekühlt wird, kondensiert der grösste Teil des in den Kondensator 59 eingeströmten Dampfes und fällt dann als Wassertropfen in die Spülkammer zurück. Der allenfalls aus dem oberen Ende des Kondensators 59 ausströmende, restliche Dampf kondensiert dann mindestens zum grössten Teil noch in den beiden Tanks 51 und 53, die ja für Luft und Dampf miteinander verbunden sind. Falls wegen der Dampfzufuhr in den beiden Wassertanks doch noch ein Überdruck entstehen sollte, wird dieser durch die erwähnte Verbindung zwischen den Tanks und der Umgebung ausgeglichen.

Nach der Desinfektion wird nochmals kurzzeitig Wasser gegen den Behälter 83 gesprüht, um diesen abzukühlen. Danach setzt das Programm-Steuerorgan 121 wieder den Motor 39 in Gang, und zwar in derjenigen Drehrichtung, in der die Türe 17 wieder geöffnet und die Halterung 27 in die Ausgangsstellung zurückgeschwenkt werden. Der gereinigte und desinfizierte Behälter 83 kann nun wieder aus der Spülkammer entnommen werden.

Eine Pflegeperson kann also den zu entleerenden und zu reinigenden Behälter in die Spülkammer einbringen und den Reinigungsvorgang starten, ohne dass sie mit ihrem in diesem Zeitpunkt von der Reinigung eines Patienten häufig schmutzigen Händen die Türe oder einen anderen Teil der Vorrichtung 5 berühren muss. Ferner wird der an der Halterung 27 befestigte Behälter, der sich bei allen Schwenkstellungen der Halterung 27 im Innern der Spülkammer befindet, ausgekippt und entleert, ohne dass dabei Schmutzteilchen oder Mikroorganismen auf die Aussenseite der Spülkammer oder gar zur Person gelangen können, die den Behälter in die Spülkammer eingesetzt hat. Dadurch wird eine grösstmögliche Hygiene erreicht.

Während des Ablaufs des Arbeitsprogramms wird durch die an der Frontwand 11 vorhandenen Lampen jeweils angezeigt, welcher Arbeitsschritt gerade stattfindet. Falls etwa der Wasser-Verdampfer überhitzt werden sollte, wird dies mit dem Temperaturfühler 81 festgestellt, der Ablauf des Programms gestoppt und mit einer der Anzeigelampen eine Störung signalisiert.

Das mit der Türe 17 verbundene Gegengewicht 25 hat ungefähr die gleichen Masse wie diese, so dass die Kette 41 durch die Masse der Türe 17 nicht oder nur wenig belastet wird. Der Motor 37 muss daher nur eine vergleichsweise kleine Leistung aufbringen und ist insbesondere so bemessen, dass er stehen bleibt, wenn etwa eine Person beim Schliessen noch den Arm oder die Hand unter der Türe halten sollte.

Da die Spülkammer-Öffnung 13b und die Türe 17 an einer vertikalen Wand der Vorrichtung angeordnet sind, braucht die obere Seite der Spülkammer für die normale Benutzung der Vorrichtung nicht zugänglich zu sein. Die Wassertanks können daher direkt über der Spülkammer angeordnet werden. Da zudem die Türe vertikal verschiebbar ist, braucht die Vorrichtung sowohl bei offener als auch bei geschlossener Türe nur relativ wenig Platz. Die parallel zur Frontwand 11 gemessene Breite kann beispielsweise ungefähr 60 cm und die rechtwinklig dazu gemessene Tiefe ungefähr 50 cm betragen.

Die zwei Wassertanks 51 und 53 gewährleisten, dass kein Wasser aus der Spülkammer in das Wasserleitungsnetz des Gebäudes zurückfliessen kann. Ferner ermöglichen sie, für die Zuleitung des Speisewassers Leitungen mit einem relativ kleinen Querschnitt zu verwenden.

Die Verwendung von zwei separaten Wassertanks, von denen der eine mit der Spül-Leitung 55 und der andere mit den Sprühdüsen 71 verbunden ist, und die Desinfektion mittels Dampf ermöglichen, mit relativ geringem Wasser- und Energieverbrauch eine optimale Reinigung und Desinfektion zu erzielen. Für die Spülung wird beispielsweise etwa 8 l Kaltwasser benötigt. Die Pumpe 67 pumpt während der Wassersprühphase etwa 20 bis 30 l Warmwasser zu den Sprühdüsen. Für die Desinfektion wird ungefähr 0,2 l Wasser verdampft.

Die Vorrichtung kann in verschiedener Hinsicht modifiziert und ausgestattet werden. Beispielsweise könnte zusätzlich zur schwenkbaren Halterung 27 in der Spülkammer noch ein unverschwenkbarer Rost oder dergleichen vorhanden sein, auf den irgendwelche Gegenstände angeordnet werden können, die gereinigt, aber nicht unbedingt gekippt werden müssen.

**Patentansprüche**

1. Verfahren zum Entleeren, Reinigen, Desinfizieren von Hygiene-Gefässen im Pflegesektor ohne Kontaminierung der äusseren Vorrichtung unter Verwendung von Wasser und Dampf, gekennzeichnet durch Aufeinanderfolge der folgenden Verfahrensstufen in einer einzigen Vorrichtung:

(a) Einführen der gefüllten, beschmutzten Gefässe in die Befestigungshalterung im Innenraum der Spülkammer der Vorrichtung,

(b) Schliessen der sich von oben nach unten senkrecht absenkenden Tür an der Vorderseite der Vorrichtung durch Tastendruck ohne Berührung mit den Händen unter gleichzeitiger Abdichtung der Spülkammer nach aussen, und gleichzeitigem Entleeren der Gefässe durch Drehung der Befestigungshalterung mit zunächst langsamer Drehungsgeschwindigkeit gegenüber der Schliessgeschwindigkeit der Tür, danach unter Steigerung der Drehungsgeschwindigkeit der schwenkbaren Befestigungshalterung bis zur synchronen Drehungsgeschwindigkeit mit der Schliessgeschwindigkeit der Tür zur praktisch vollständigen Entleerung mittels eines elipsenförmigen Kettenrades auf der Drehachse der Befestigungshalterung über eine Kette,

(c) Grobschmutzentfernung durch Einführung von drucklosem Kaltwasser aus einem, sich über der Spülkammer befindenden, ersten Behälter über eine, sich im oberen Teil der Spülkammer befindende Sprühdüse mittels tangential auf die innere Wandung auftreffenden Wasserstrahles zur vollständigen Entfernung der flüssigen und/oder festen Verschmutzung durch die sich ausbildende Rotationsfläche des Wasserstrahles in Drehrichtung des Auftreffwinkels aus dem unteren, konischen Behälterteil über den Abschlusssyphon,

(d) Gefäss- und Spülkammer-Reinigung durch warmes Mischwasser aus einem sich über der Spülkammer befindenden zweiten Behälter mittels Druckpumpe über an der Decke, den Seitenwänden und im unteren, konischen Teil der Sprühkammer sich befindende Sprühdüsen, derart, dass der geteilte Druckwasserstrahl strahlungsgeometrisch den Innenraum der Spülkammer und die Wände der Spülkammer in Teilvolumina durch die einzelnen Sprühdüsen gleichmässig verteilt und in gleichen Mengen in der Zeiteinheit besprüht und dadurch die vollständige Reinigung aller Flächen kurzzeitig mit optimal geringer Wassermenge erfolgt,

(e) Desinfektion der entleerten gereinigten Gefässe und des Innenraumes der Spülkammer durch heissen Wasserdampf aus einem, sich im unteren Teil der Vorrichtung befindenden Dampfgenerator, dessen Aufheizung bereits beim Schliessen der Tür nach Stufe (b) beginnt, derart, dass der Heissdampf durch alle Druckdüsen der Stufe (d) in gleicher Richtung und gleicher Verteilung in die Sprühkammer eintritt, und die gleichmässige Erhitzung der Gefässe und des Innenraumes der Spülkammer erfolgt,

(f) Kondensation des Wasserdampfes zeitlich gleichzeitig zur Desinfektion in Stufe (e) zur Bewirkung einer dynamischen Durchströmung des Innenraumes der Sprühkammer in Stufe (e) durch Austritt des Wasserdampfes aus einem Rohr oberhalb der Wasseroberfläche in den ersten Behälter der Stufe (c) unter mehrfacher Umlenkung der Strömungsrichtung des Wasserdampfes zur Verringerung der Strömungsgeschwindigkeit und der Dampfmenge zur kontinuierlichen Kondensation des Wasserdampfes in Fliessrichtung an den schräg stehenden, nach unten gerichteten Kondensationslamellen auf der Wasseroberfläche des Behälters unter Vermeidung des Austritts von Wasserdampf aus der Tür,

(g) Restkondensation der Dampfbrüden und Rückkühlung der Gefässe und der Spülkammer durch Einführen von Druckwasser mittels der Druckpumpe über alle Sprühdüsen nach Stufe (d), Ablauf des Restwassers aus den Leitungen und Düsen durch den unteren, konischen Teil der Sprühkammer über den Syphon,

(h) Öffnen der Tür nach oben unter gleichzeitiger Drehung der Befestigungshalterung in umgekehrter Drehrichtung zum Drehvorgang nach Stufe (b), Entnahme der leeren, gereinigten, desinfizierten Gefässe, Wiederholung der Verfahrensstufen zur periodischen, fortlaufenden Entleerung, Reinigung, Desinfektion.

(i) Steuerung der Massnahmen der Stufen (b) bis (h) nach Einleiten der Stufe (a) durch Programmsteuerung.

2. Verfahren nach Anspruch 1, dadurch gekennzeichnet, dass die Drehung der Befestigungshalterung in Stufe (b) beim Schliessen der Tür so langsam erfolgt, dass ein Austreten der Gefässinhalte in Richtung der Tür vermieden ist, und die vollständige Entleerung erst bei geschlossener Tür mit der Gefässöffnung nach unten bis zur waagerechten Lage erfolgt.

3. Verfahren nach den Ansprüchen 1 und 2, dadurch gekennzeichnet, dass das Mischwasser in Stufe (d) eine Temperatur von 40° bis 45°C aufweist und mit einem Wasserdruck von 1,75 bar bei etwa 50 l/min, oder 1,90 bar bei etwa 34 l/min, oder 2,50 bar bei etwa 22 l/min durch die Sprühdüsen eingeführt wird.

4. Verfahren nach den Ansprüchen 1 und 3, dadurch gekennzeichnet, dass die Sprühzeit in Stufe (d) 30 bis 35 sec beträgt.

5. Verfahren nach den Ansprüchen 1 bis 4, dadurch gekennzeichnet, dass der Wasserdampf in Stufe (e) heisser Nassdampf ist, der bei 420 mm WS Druck eine Temperatur von 98°C bis 100°C aufweist.

6. Verfahren nach den Ansprüchen 1 bis 5, dadurch gekennzeichnet, dass die Desinfektionszeit etwa 60 bis 70 sec beträgt.

7. Verfahren nach den Ansprüchen 1 bis 6, dadurch gekennzeichnet, dass in Stufe (f) die Kondensationsgeschwindigkeit durch die Anzahl der Kondensationslamellen, oder durch den Winkel der Schrägstellung, oder durch den Abstand des unteren Endes von der Wasseroberfläche geregelt wird.

8. Verfahren nach den Ansprüchen 1 bis 7, dadurch gekennzeichnet, dass das Ansteigen der Wassermenge im unteren, konischen Teil des Sprühraumes der Vorrichtung in Stufe (d) bei einer Störung der Zeitsteuerung mittels eines Differenzdruckmessers der in einem Raum befindlichen Luftsäule unterbrochen wird.

9. Verfahren nach den Ansprüchen 1 bis 7, dadurch gekennzeichnet, dass bei Nichterreichen der Temperatur des Wasserdampfes in Stufe (e) durch eine Störung, ein sich in dem Aussenraum der Spülkammer befindlicher, durch Bimetall gesteuerter Thermostat den Vorgang der Desinfektion unterbricht.

10. Vorrichtung zum Spülen und Desinfizieren von Behältern, insbesondere Steckbecken und Urinflaschen, nach dem Verfahren der Ansprüche 1 bis 7, mit einer mit Sprühdüsen (71) versehenen Spülkammer (13), die auf einer Seite eine, durch eine Tür (17) verschliessbare Öffnung (13b) aufweist, und mindestens einer zum Halten und Auskippen eines Behälters (83) dienenden, um eine horizontale Schwenkachse (29) schwenkbaren Halterung (27), dadurch gekennzeichnet, dass

(a) ein Motor (37) vorhanden ist, der mit einem Kettenrad (39) verbunden ist, das in eine Kette (41) eingreift, die einen vertikal auf- und abbewegbaren Abschnitt aufweist, der mit der entlang einer Vertikalebene verschiebbar geführten Tür (17) verbunden ist, um diese zu öffnen und zu schliessen, und gleichzeitig die Halterung (27), die derart in der Spülkammer (13) angeordnet ist, dass sie sich in jeder Schwenkstellung im Innern der Spülkammer (13) befindet, und die drehfest mit einem, in die gleiche Kette (41) eingreifenden, ellipsenförmigen Kettenrad (31) verbunden ist, zu schwenken,

(b) ein mindestens zum Teil oberhalb der Spülkammer (13) angeordneter und mit einer Kaltwasser-Speiseleitung (87) verbundener Wassertank (51) vorhanden ist, der mit einer, ein Ventil (57) enthaltenden, getrennt von den Sprühdüsen (71) in die Spülkammer (13) mündenden Spülleitung (55) verbunden ist, die derart ausgebildet ist, dass bei geöffnetem Ventil (57) Wasser infolge der Schwerkraft aus dem Wassertank so in die Spülkammer (13) strömt, dass es tangential auf die innere Wandung auftrifft,

(c) zusätzlich zu diesem Wassertank (51) ein anderer Wassertank (53) vorhanden ist, der über eine Pumpe (67) mit den Sprühdüsen (71), mit einer Kaltwasser-Speiseleitung (87) und einer Heisswasser-Speiseleitung (103) verbunden ist,

(d) die Spülkammer (13) mit einem Dampfkondensator (59) verbunden ist, der in dem, mit der Spülleitung (55) verbundenen Wassertank (51) angeordnet ist und schrägstehende, nach unten auf die Wasseroberfläche weisende Kondensationslamellen aufweist,

(e) ein Programmsteuerungsorgan (121) zum Steuern des Arbeitsablaufes vorhanden ist,

(f) ein Wasserverdampfer (77) vorhanden ist, der mit einer Verdampferkammer (77a) und einem, durch das Programm-Steuerorgan (121) ein- und ausschaltbaren Heizelement (79) ausgestattet ist,

(g) die Verdampferkammer (77a) mit einer, die Pumpe (67) mit den Sprühdüsen (71) verbindenden Leitung (69) verbunden ist, wobei die Verbindung zwischen der Verdampferkammer (77a) und der Pumpe (67) sowie den Sprühdüsen (71) dauernd offen ist,

(h) sich die Pumpe (67) unterhalb des Füllniveaus (63) des mit ihr verbundenen Wassertanks (53) befindet,

(i) sich die Sprühdüsen (71) unterhalb der Pumpe (67) und der Wasserverdampfer (77) unterhalb der Sprühdüsen (71) befinden,

(j) die Leitung (69) zwischen der Pumpe (67) und den Sprühdüsen (71) einen, sich oberhalb des Füllniveaus (63) des mit der Pumpe (67) verbundenen Wassertanks (53) befindenden Abschnitt (69b) aufweist.

## Claims

1. Method for the emptying, cleaning, disinfecting of hygiene receptacles in the nursing sector, without contamination of the external device using water and steam, characterized by the sequence of the following process steps in a single device:

(a) introduction of the filled, soiled receptacles into the mounting support in the interior of the rinsing chamber of the device,

(b) closing of the door, which drops vertically from above downwards on the front side of the device through the pushing of a button without touching by hand, with simultaneous sealing of the rinsing chamber externally, and simultaneous emptying of the receptacles through rotation of the mounting support, firstly with a slow rotational speed compared with the closing speed of the door, then with an increase in the rotational speed of the pivotable mounting support until a synchronous rotational speed with the closing speed of the door is reached for practically complete emptying, by means of a ellipse-shaped chain wheel on the rotational axis of the mounting support by way of a chain,

(c) coarse dirt removal through introduction of pressureless cold water from a first container situated over the rinsing chamber via a spraying nozzle situated in the upper part of the rinsing chamber by means of a water jet striking the inner wall tangentially for the complete removal of the fluid and/or solid dirt through the surface of revolution of the water jet forming in the direction of rotation of the angle of impact from the lower, conical part of the container via the seal syphon,

(d) receptacle- and rinsing chamber-cleaning by warm mixed water from a second container situated over the rinsing chamber, by means of a pressure pump via spraying nozzles situated on the cover, the side walls and in the lower, conical part of the spraying chamber, such that the divided spraying water jet geometrically sprays the interior of the rinsing chamber and the walls of the rinsing chamber in part volume through the individual spraying nozzles evenly distributed and in equal quantities per unit time and thereby the complete cleaning of all surfaces in a short time with an optimally minimum quantity of water takes place,

(e) disinfection of the emptied, cleaned receptacles and of the interior of the rinsing chamber by hot steam from a steam generator situated in the lower part of the device, the heating of which already begins on the closing of the door after step (b), such that the hot steam enters through all pressure nozzles of step (d) in the same direction and with the same distribution into the spraying chamber, and the uniform heating of the receptacles and of the interior of the rinsing chamber takes place,

(f) condensation of the steam per unit time simultaneously with the disinfection in step (e) to cause a dynamic through flow of the interior of the spraying chamber in step (e) through outlet of the steam from a pipe above the water surface into the first container of step (c) with multiple reorientation of the direction of flow of the steam to reduce the speed of flow and the quantity of steam for the continuous condensation of the steam in the direction of flow against the condensation plates, which stand obliquely and are directed downward, on the water surface of the container, avoiding the outlet of steam out of the door,

(g) residual condensation of the steam vapours and recooling of the receptacles and rinsing chamber through introduction of pressure water by means of the pressure pump via all spraying nozzles following step (d), outlet of the residual water out of the pipes and nozzles through the lower, conical part of the spraying chamber via the syphon,

(h) opening of the door upwards with simultaneous rotation of the mounting support in reverse direction of rotation to the rotation process following step (b), removal of the empty, cleaned, disinfected receptacles, repetition of the process steps for the recurrent, continuous removal, cleaning, disinfection.

(i) controlling of the measures of steps (b) to (h) after commencement of step (a) by program control.

2. Method according to Claim 1, characterized in that the rotation of the mounting support in step (b) on closing of the door takes place so slowly that an outlet of the contents of the receptacle towards the door is avoided, and the complete emptying takes place only with a closed door with the receptacle opening downward to the horizontal position.

3. Method according to Claims 1 and 2, characterized in that the mixed water in step (d) has a temperature of 40° to 45°C and is introduced at a water pressure of 1.75 bar at approximately 50 l/min, or 1.90 bar at approximately 34 l/min, or 2.50 bar at approximately 22 l/min through the spraying nozzles.

4. Method according to Claims 1 and 3, characterized in that the spraying time in step (d) amounts to 30 to 35 sec.

5. Method according to Claims 1 to 4, characterized in that the steam in step (e) is hot wet steam, which at 420 mm water-column pressure has a temperature of 98°C to 100°C.

6. Method according to Claims 1 to 5, characterized in that the disinfection time amounts to approximately 60 to 70 sec.

7. Method according to Claims 1 to 6, characterized in that in step (f) the condensation speed is regulated by the number of condensation plates, or by the angle of inclination, or by the distance of the lower end from the water surface.

8. Method according to Claims 1 to 7, characterized in that the rise of the quantity of water in the lower, conical part of the spraying chamber of the device in step (d) is interrupted on a disturbance of the time control by means of a differential pressure gauge of the column of air situated in a chamber.

9. Method according to Claims 1 to 7, characterized in that when the temperature of the steam in step (e) is not reached through a breakdown a thermostat situated in the outer area of the rinsing chamber, controlled by bimetal, interrupts the process of disinfection.

10. Device for the rinsing and disinfection of containers, in particular bedpans and urine bottles, according to the method of Claims 1 to 7, with a rinsing chamber (13) provided with spraying nozzles (71), which on one side has an opening (13b) which is able to be closed by a door (17), and at least one mounting support (27) pivotable about a horizontal swivel axis (29) serving to hold and tip out a container (83), characterized in that

(a) a motor (37) is present, which is connected with a chain wheel (39), which engages into a chain (41), which has a section movable vertically up and down, which is connected with the door (17) slidably guided along a vertical plane, in order to open and close this, and at the same time to pivot the mounting support (27), which is arranged in the rinsing chamber (13) such that in each pivot position it is situated in the interior of the rinsing chamber (13), and which is connected firmly against rotation with an ellipse-shaped chain wheel (31) engaging into the same chain (41),

(b) a water tank (51) is present, arranged at least partly above the rinsing chamber (13) and connected with a cold water supply pipe (87), and is connected with a rinsing pipe (55) opening into the rinsing chamber (13) separately from the spraying nozzles (71) and containing a valve (57), and which constructed such that with an opened valve (57) water flows out of the water tank, as a result of gravity, such that it tangentially hits the inner wall,

(c) in addition to this water tank (51) another water tank (53) is present, which is connected by way of a pump (67) with the spraying nozzles (71), with a cold water supply pipe (87) and a hot water supply pipe (103),

(d) the rinsing chamber (13) is connected with a steam condenser (59), which is arranged in the water tank (51) connected with the rinsing pipe (55) and has inclined condensation plates facing downwards towards the water surface,

(e) a program control element (121) to control the operating sequence is present,

(f) a water evaporator (77) is present, which is fitted with an evaporator chamber (77a) and with a heating element (79) which is able to be switched on and off by the program control element (121),

(g) the evaporator chamber (77a) is connected with a pipe (69) connecting the pump (67) with the spraying nozzles (71), whereby the connection between the evaporator chamber (77a) and the pump (67), and also the spraying nozzles (71), is permanently open,

(h) the pump (67) is situated beneath the filling level (63) of the water tank (53) connected therewith,

(i) the spraying nozzles (71) are situated beneath the pump (67) and the water evaporator (77) is situated beneath the spraying nozzles (71),

(j) the pipe (69) between the pump (67) and the spraying nozzles (71) has a section (69b) situated above the filling level (63) of the water tank (53) connected with the pump (67).

## Revendications

1. Procédé pour vider, pour nettoyer et pour désinfecter des récipients d'hygiène dans le domaine sanitaire, sans contamination du dispositif extérieur et en utilisant de l'eau et de la vapeur, caractérisé par la succession des stades de procédés suivants dans un appareillage unique:

(a) introduction des récipients pleins salis dans le support de fixation à l'intérieur de la chambre de lavage de l'appareillage,

(b) fermeture de la porte s'abaissant verticalement du haut vers le bas sur le côté antérieur de l'appareillage, en appuyant sur une touche, sans que les mains viennent en contact avec la porte, en rendant simultanément étanche la chambre de lavage vis-à-vis de l'extérieur, et vidange simultanée des récipients par rotation du support de fixation, tout d'abord à une vitesse de rotation plus lente que la vitesse de fermeture de la porte, puis en augmentant la vitesse de rotation du support de fixation pivotant jusqu'à ce que la vitesse de rotation soit en synchronisme avec la vitesse de fermeture de la porte, pour obtenir une vidange pratiquement complète au moyen d'une roue à chaîne en forme d'ellipse montée sur l'axe de rotation du support de fixation par l'intermédiaire d'une chaîne,

(c) enlèvement grossier des saletés par introduction d'eau froide qui n'est pas sous pression et qui provient d'un premier récipient se trouvant au-dessus de la chambre de lavage, par l'intermédiaire d'une buse de projection se trouvant dans la partie supérieure de la chambre de lavage, au moyen d'un jet d'eau arrivant tangentiellement sur la paroi intérieure, pour enlever entièrement la saleté liquide et/ou solide par la partie inférieure conique du récipient par l'intermédiaire du siphon d'évacuation, grâce à la surface de révolution du jet d'eau qui se forme suivant le sens de rotation de l'angle d'incidence,

(d) nettoyage du récipient et de la chambre de lavage par de l'eau chaude de mélange provenant d'un second récipient se trouvant au-dessus de la chambre de lavage, au moyen d'une pompe de refoulement, par l'intermédiaire de buses de projection se trouvant sur le sommet, sur les parois latérales et sur la partie inférieure conique de la chambre de projection, de manière à ce que le jet d'eau sous pression subdivisé divise de manière régulière, suivant une géométrie de projection, par les diverses buses de projection, l'intérieur de la chambre de lavage et les parois de la chambre de lavage en volumes partiels, et soit projeté en de mêmes quantités par unité de temps, en provoquant le nettoyage complet de toutes les surfaces en peu de temps et avec une quantité optimale d'eau qui est petite,

(e) désinfection des récipients vidés et nettoyés et de l'intérieur de la chambre de lavage par de la vapeur d'eau chaude provenant d'un générateur de vapeur se trouvant dans la partie inférieure de l'appareillage, dont le chauffage a commencé dès la fermeture de la porte, après le stade (b), de manière à ce que la vapeur d'eau surchauffée entre dans la chambre de projection suivant la même direction et avec la même répartition, par toutes les buses de projection sous pression du stade (d), et de manière à provoquer le chauffage uniforme des récipients et de l'intérieur de la chambre de lavage,

(f) condensation temporaire de la vapeur d'eau en même temps que la désinfection du stade (e) pour provoquer une traversée dynamique de l'intérieur de la chambre de lavage au stade (e) par sortie de la vapeur d'eau d'un tube débouchant au-dessus de la surface libre de l'eau dans le premier récipient du stade (c), avec de nombreuses déviations de la direction d'écoulement de la vapeur d'eau, afin de diminuer la vitesse d'écoulement et la quantité de vapeur en vue de la condensation continue de la vapeur d'eau sur des lamelles de condensation inclinées dans le sens de l'écoulement et dirigées vers le bas sur la surface libre de l'eau du récipient, en évitant la sortie de vapeur d'eau par la porte,

(g) condensation des buées de vapeur restantes et refroidissement des récipients et de la chambre de lavage par envoi d'eau sous pression au moyen de la pompe de refoulement, par l'intermédiaire de toutes les buses de projection suivant le stade (d), évacuation de l'eau restante dans les conduits et les buses par la partie inférieure conique de la chambre de projection, par l'intermédiaire du siphon,

(h) ouverture de la porte vers le haut avec rotation simultanée du support de fixation dans le sens de rotation opposé à celui suivant le stade (b), enlèvement des récipients vides nettoyés et désinfectés, répétition des stades du procédé pour vider, nettoyer et désinfecter d'une manière suivie, périodiquement,

(i) commande des opérations des stades (b) à (h) après que le stade (a) a commencé, par commande par programme.

2. Procédé suivant la revendication 1, caractérisé en ce qu'il consiste à effectuer la rotation du support de fixation au stade (b), lors de la fermeture de la porte, si lentement que toute sortie du contenu du récipient dans la direction de la porte est empêchée et que la vidange complète ne s'effectue que lorsque la porte est fermée avec l'ouverture du récipient dirigée vers le bas, jusqu'à la position horizontale.

3. Procédé suivant les revendications 1 et 2, caractérisé en ce que l'eau de mélange au stade (b) a une température de 40° à 45°C et est introduite par les buses de projection sous une pression de 1,75 bar pour un débit de 50 l/min environ, ou de 1,90 bar pour un débit de 34 l/min environ, ou de 2,50 bars pour un débit de 22 l/min environ.

4. Procédé suivant les revendications 1 et 3, caractérisé en ce que la durée de projection au stade (d) est comprise entre 30 à 35 secondes.

5. Procédé suivant les revendications 1 à 4, caractérisé en ce que la vapeur d'eau au stade (e) est de la vapeur humide chaude qui, pour une pression correspondant à une colonne d'eau de 420 mm, a une température de 98°C à 100°C.

6. Procédé suivant les revendications 1 à 5, caractérisé en ce que la durée de désinfection est comprise entre 60 et 70 secondes environ.

7. Procédé suivant les revendications 1 à 6, caractérisé en ce qu'au stade (f) la vitesse de condensation est réglée par le nombre des lamelles de condensation ou par l'angle d'inclinaison, ou par la distance entre l'extrémité inférieure et la surface libre de l'eau.

8. Procédé suivant les revendications 1 à 7, caractérisé en ce que l'ascension de la quantité d'eau dans la partie inférieure conique de la chambre de projection de l'appareillage au stade (d) est interrompue, dans le cas d'un dérangement de la commande en synchronisation, au moyen d'un manomètre différentiel mesurant la colonne d'air se trouvant dans une chambre.

9. Procédé suivant les revendications 1 à 7, caractérisé en ce que, dans le cas où l'on n'atteint pas la température de la vapeur d'eau au stade (e), en raison d'un dérangement, un thermostat, commandé par un bilame et se trouvant à l'extérieur de la chambre de lavage, interrompt le processus de désinfection.

10. Appareillage pour laver et pour désinfecter des récipients, notamment des bassins de lit et des flacons à urine, suivant le procédé des revendications 1 à 7, comprenant une chambre de lavage (13) munie de buses de projection (71), qui est munie d'un côté d'une ouverture (13b) pouvant être fermée par une porte (17) et d'au moins un support (27) pivotant autour d'un axe horizontal (29) et servant à retenir et à faire basculer un récipient (83), caractérisé en ce que:

(a) il est prévu un moteur (37) qui est relié à une roue à chaîne (39) engrenant dans une chaîne (41) qui présente un tronçon vertical, allant et venant, qui est relié à la porte (17) guidée de manière à pouvoir se déplacer le long d'un plan vertical, afin d'ouvrir et de fermer celle-ci et, en même temps, à faire pivoter le support (27) qui est disposé dans la chambre de lavage (13) de manière à se trouver à l'intérieur de la chambre de lavage (13) en toute position de pivotement et y est solidaire en rotation d'une roue à chaîne (31) de forme ellipsoïdale engrenant dans la même chaîne (41),

(b) il est prévu un réservoir à eau (51) disposé au moins en partie au-dessus de la chambre de lavage (13) et communiquant avec un conduit (87) d'alimentation en eau froide, ce récipient (51) communiquant avec un conduit de lavage (55) sur lequel est montée une vanne (57) et qui débouche dans la chambre de lavage (13) séparément des buses de projection (71) de manière à ce que, lorsque la vanne (57) est ouverte, de l'eau s'écoule, en raison de la force de gravité, du réservoir à eau dans la chambre de lavage (13) en arrivant tangentiellement sur la paroi intérieure,

(c) en plus de ce réservoir à eau (51) est prévu un autre réservoir à eau (53), qui communique, par une pompe (67) avec les buses de projection (71), avec un conduit (87) d'alimentation en eau froide et avec un conduit (103) d'alimentation en eau chaude,

(d) la chambre de lavage (13) communique avec un condenseur de vapeur (59), qui est disposé dans le réservoir à eau (51) communiquant avec le conduit de lavage (55) et qui présente des lamelles de condensation inclinées dirigées vers le bas sur la surface libre de l'eau,

(e) il est prévu un organe de commande par programme (121) pour commander le déroulement des opérations,

(f) il est prévu un évaporateur pour l'eau (77), qui est équipé d'une chambre formant évaporateur (77a) et d'un élément de chauffage (79) pouvant être branché et débranché par l'organe de commande par programme (121),

(g) la chambre formant évaporateur (77a) communique avec un conduit (69) mettant en communication la pompe (67) avec les buses de projection (71), la communication entre la chambre formant évaporateur (77a) et la pompe (67), ainsi qu'avec les buses de projection (71) étant ouverte en permanence,

(h) la pompe (67) se trouve en-dessous du niveau de remplissage (63) du réservoir à eau (53) avec lequel elle communique,

(i) les buses de projection (71) se trouvent en-dessous de la pompe (67) et l'évaporateur pour l'eau (77) se trouve en-dessous des buses de projection (71),

(j) le conduit (69) entre la pompe (67) et les buses de projection (71) présente un tronçon (69b) se trouvant au-dessus du niveau de remplissage (63) du réservoir à eau (53) communiquant avec la pompe (67).

# Fig. 1

# Fig. 2

0 047 408

Fig.3

17

Fig. 4

0 047 408

19